# EUROPÄISCHE PATENTSCHRIFT

(11) **EP 0 599 065 B1**
(45) Veröffentlichungstag und Bekanntmachung des Hinweises auf die Patenterteilung: **17.09.1997**
(21) Anmeldenummer: 93117210.0
(22) Anmeldetag: 23.10.1993
(51) Int. Cl.: A61M 25/00, A61F 2/06

(54) **Rohrförmige Ureterschiene**
Tubular ureter splint
Conduit urétéral tubulaire

(30) Priorität: 29.10.1992 DE 4236571
(43) Veröffentlichungstag der Anmeldung: 01.06.1994
(73) Patentinhaber: Sachse, Hans, Professor Dr., 90425 Nürnberg (DE)
(72) Erfinder: Sachse, Hans, Professor Dr., 90425 Nürnberg (DE)
(74) Vertreter: Richter, Bernhard, Dipl.-Ing.

(56) Entgegenhaltungen:
- EP-A- 0 018 179
- EP-A- 0 086 573
- EP-A- 0 351 864
- DE-A- 4 028 477
- GB-A- 1 088 403
- US-A- 4 177 814
- US-A- 4 828 554
- US-A- 5 005 732
- US-A- 5 085 635

## Beschreibung

Die Erfindung geht zunächst aus von einer rohrförmigen Ureterschiene aus einem elastischen Material, insbesondere Kunststoff, die in ihrer Gebrauchslage mit einem ihrer beiden Enden im Nierenbecken und mit ihrem anderen Ende in der Blase gelegen ist, wobei an das zum Einbringen in die Blase vorgesehenen Ende außenseitig ein dünnwandiger, weicher und zumindest in seinem Querschnitt einstückiger Schlauch dicht angebracht ist, der über die Stirnfläche dieses Ureterschienenendes hinausragt (Oberbegriff des Anspruches 1). Eine solche Ureterschiene ist aus DE-OS 40 28 477 bekannt. Hierzu ist es ferner bekannt und auch der vorgenannten Literaturstelle zu entnehmen, daß zweckmäßigerweise die Ureterschiene an ihren Enden eine eingeprägte Krümmung (sogenannte "Memory") aufweist, mit der diese Enden sich sowohl im Nierenbecken, als auch in der Blase halten, so daß sich die Ureterschiene nicht ungewollt in ihrer Längsrichtung im Ureter verlagern kann. Mit der Ureterschiene gemäß DE-OS 40 28 477 wird zwar verhindert, daß bei einer Drucksteigerung in der Blase der Urin in umgekehrter Richtung von der Blase her zum Nierenbecken hochgedrückt wird und dadurch eine funktionelle Störung und Schädigung des Nierengewebes auslöst. Der dieser Literaturstelle zu entnehmende kreisrunde Querschnitt des Schlauches gibt ihm aber eine hier unerwünschte Stabilität gegen die angestrebte Formänderung des Schlauches, welche den Verschluß des blasenseitigen Ureterschienenendes bewirkt und damit das vorgenannte Hochsteigen des Urins verhindert.

Ferner ist es aus DE-OS 35 25 165 bekannt, durch ein Rückschlagventil in Form eines Folienventiles das unerwünschte Hochsteigen des Urins von der Blase ins Nierenbecken zu verhindern. Das Folienventil besteht dabei aus zwei flachen, dünnwandigen Folien, die an ihren seitlichen Rändern miteinander verklebt oder verschweißt sind. Hierbei besteht der Nachteil, daß innenseitig in den Winkeln neben den Schweißstellen sich Inkrustationen bilden. Die vorgenannten Verschweißungen und die zwischen ihnen bestehenden innenseitigen Winkel verhindern, zumindest erschweren sehr das dichte Anbringen eines solchen Rückschlagventiles an der Außenseite der Ureterschiene. Das Herstellen eines solchen Folienventiles ist gegenüber dem Herstellen eines in sich einstückigen Schlauches mit größeren Kosten verbunden. Außerdem können solche Schweißnähte unter ungünstigen Umständen undicht werden. Eine solche Anordnung erfüllt im übrigen nicht den o.g. Oberbegriff des Anspruches 1.

Die Aufgabenstellung der Erfindung besteht zunächst darin, eine Anordnung gemäß DE-OS 40 28 477 dahingehend zu verbessern, daß auch bei einer sehr geringen Drucksteigerung im Blaseninnern mit Hilfe des Schlauches das Hochsteigen des Urins vehindert wird.

Zur Lösung dieser Aufgabe ist zunächst, ausgehend von einer Ureterschiene gemäß dem Oberbegriff des Anspruches 1 vorgesehen, daß der Querschnitt des Schlauches unrund ist (Kennzeichen des Anspruches 1). Mit einer solchen Querschnittsgebung hat man einerseits die Vorteile geringer Fertigungskosten und großer Stabilität eines einstückigen und daher mit Sicherheit dichten Schlauches mit dem Vorteil verbunden, daß aufgrund der Unrundheit des Querschnittes ein solcher Schlauch sich schon bei einem geringeren Druckgefälle zusammenlegt und damit die Abdichtung der Ureterschiene gegen das Hindurchtreten von Harn bewirkt, als es bei einem im Querschnitt kreisrunden Schlauch der Fall wäre. Die Ansprüche 2 und 3 stellen bevorzugte Ausführungsformen des Anspruches 1 dar. Das vorgenannte Druckgefälle entsteht dadurch, daß bei einer Drucksteigerung in der Blase eine minimale Menge Urin durch die Ureterschiene nierenwärts fließt. Dadurch entsteht ein Druckgefälle zwischen Ureterinnenlumen und Blase. Dieses Druckgefälle bewirkt, daß sich der Schlauch (auch Antirefluxschlauch genannt) vor die blasenseitigen Öffnungen, insbesondere das Innenlumen der Ureterschiene, legt und dadurch den weiteren Eintritt von Urin in die Ureterschiene und damit zum Nierenbecken verhindert.

Die eingangs genannte Aufgabe kann ferner durch die Lehre des Anspruches 4 gelöst werden. Dieser Anspruch 4 ist in seinem Oberbegriff identisch mit dem eingangs angeführten Oberbegriff des Anspruches 1 und sieht in seinem Kennzeichen vor, daß der die Stirnfläche des blasenseitigen Ureterschienenendes überragende Teil des Schlauches eine eingeprägte Form aufweist derart, daß der Schlauch eine Zick-Zack-Form, oder eine Spiralform, oder eine Korkenzieherform aufweist, oder daß sich entlang des überragenden Schlauchabschnittes dessen Durchmesser vergrößert und wieder verkleinert. Hiermit legt sich der Schlauch bei der o.g. Erhöhung des Innendruckes entsprechend seiner jeweils ihm eingeprägten Form in seiner Längsrichtung betrachtet zusammen. Dies hat zur Folge, daß sich die Schlauchwandungen aneinander und auch gegen das blasenseitige Austrittsende des Innenlumens der Ureterschiene legen. Auch hierdurch wird bei nur sehr geringer Drucksteigerung im Blaseninnern das Abdichten des blasenseitigen Ureterschienenendes gegen das Hochsteigen des Urins erreicht. Dieser Vorteil kann vom Prinzip her schon bei einem Schlauch mit kreisrundem Querschnitt erreicht werden. Es ist aber eine bevorzugte Ausführung der Erfindung, wenn gemäß Anspruch 5 die Lehren der Ansprüche 1 und 4 zusammengefaßt werden. Dabei kann in der bevorzugten Ausführung gemäß Anspruch 6 die Lage der langen Achse des unrunden Querschnittes so sein, daß dies das Aufeinanderlegen und Abdichten der zick-zack-förmigen, spiralförmigen, korkenzieherförmigen oder sich im Querschnitt ändernden Wandteile des Schlauches aufeinander im Falle einer Drucksteigerung im Blaseninnern erleichtert.

Die Merkmale des Anspruches 8 sind zwar für sich aus DE-OS 40 28 477 bekannt. Sie stellen aber im Rahmen der vorliegenden Erfindung vorteilhafte Merkmale dar.

Die Merkmale des Anspruches 10 ermöglichen auch bei einer Anordnung nach Anspruch 4 das Einbringen eines Mandrins.

Weitere Vorteile und Merkmale der Erfindung sind den übrigen Unteransprüchen, sowie der nachfolgenden Beschreibung und der zugehörigen Zeichnung von erfindungsgemäßen Ausführungsmöglichkeiten zu entnehmen. In der Zeichnung zeigt:
- Fig. 1:: eine erste Ausführungsform der Erfindung und zugleich die prinzipielle Anordnung einer solchen Ureterschiene,
- Fig. 2 und 3:: verschiedene Ausführungen der Erfindung im Schnitt der Linie A-B in Fig. 1,
- Fig. 4:: eine weitere Ausführungsform der Erfindung im Schnitt und in der Abdichtstellung,
- Fig. 4a:: einen Schnitt gemäß der Linie E-F in Fig. 4,
- Fig. 5:: die Ausführung nach Fig. 4 in der Offenstellung, ebenfalls im Schnitt,
- Fig. 6:: eine weitere Ausführung der Erfindung in der Seitenansicht,
- Fig. 7:: eine andere Ausführung der Erfindung, ebenfalls in der Seitenansicht,
- Fig. 8:: einen Schnitt nach der Linie C-D in Fig. 7,
- Fig. 9:: eine weitere Ausführung der Erfindung, ebenfalls im Schnitt.

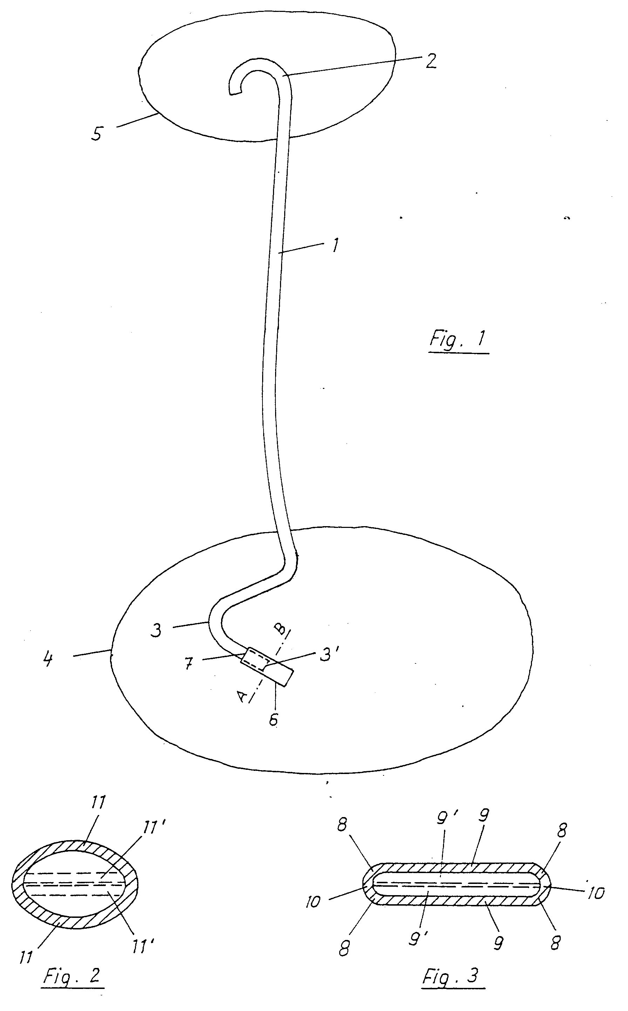

Fig. 1 zeigt prinzipiell eine Ureterschiene 1 mit nierenseitigem Ende 2 und blasenseitigem Ende 3, die in der Regel mit einer eingeprägten Krümmung (sogenannter "Memory") versehen sind. Nach Einführen der Ureterschiene in die Blase 4 und von dort durch den nicht dargestellten Ureter in das Becken der Niere 5 halten die gekrümmten Enden 2, 3 die Ureterschiene 1 in dieser Arbeitsposition. Das blasenseitige Ende 3 ist mit einem dünnwandigen, weichen Schlauch 6 versehen, der bei Ansteigen des Druckes in der Blase sich so zusammenlegt, daß er ein Hochsteigen des in der Blase 4 befindlichen Urins durch die Ureterschiene 1 in die Niere verhindert. Nachstehend sind mehrere Ausführungsmöglichkeiten der Gestaltung dieses Schlauches beschrieben. Der Schlauch 6 kann gemäß Fig. 1 vom Ende 3 her schräg nach unten gerichtet sein. Er kann aber auch eine andere Richtung aufweisen.

Der Schlauch 6 ist zumindest in seinem Querschnitt, bevorzugt aber auch zugleich über seine Länge einstückig. Er ist über das blasenseitige Ende 3 der Ureterschiene 1 geschoben und an seinem Umfang gemäß Ziffer 7 dicht an der Außenfläche der Ureterschiene 3 angebracht, z.B. durch Verkleben oder Verschweißen, insbesondere durch Heißverschweißen. Der Schlauch 6 hat gemäß der Schnittlinie A-B einen unrunden Querschnitt, der beispielsweise oval oder elliptisch sein kann (Fig. 2) oder aber gemäß Fig. 3 langgestreckt ist und etwa die Form eines länglichen Rechteckes hat. Aus Fertigungsgründen sind dabei die Übergangskanten 8 von den langen Seiten 9 zu den kurzen Seiten 10 des Querschnittes abgerundet. Aufgrund der Formbarkeit und gegebenenfalls auch Elastizität des Schlauchmaterials kann der Schlauch 6 auch mit einem unrunden Querschnitt über die im Querschnitt runde Ureterschiene geschoben und daran gemäß Erläuterung zu Ziffer 7 befestigt werden.

Füllt sich die Blase so weit mit Harn, daß der in ihr entstehende Druck den Harn durch die Ureterschiene nach oben drücken kann, so bewirkt das eingangs erläuterte Druckgefälle ein Kollabieren des Schlauches derart, daß die Wandungen 9 gemäß Fig. 3 und die entsprechenden langen Wandungen 11 des Querschnittes nach Fig. 2, wie gestrichelt angedeutet und mit 9', 11' beziffert, aneinander dichtend anliegen. Hiermit wird das schädliche Hochsteigen des Harns in das Nierenbecken verhindert. Bei gewünschtem normalen Verhalten dagegen wird der Harn aus der Blase vom Patienten abgelassen. Das vorgenannte Druckgefälle entsteht nicht und der Schlauch 6 ist offen.

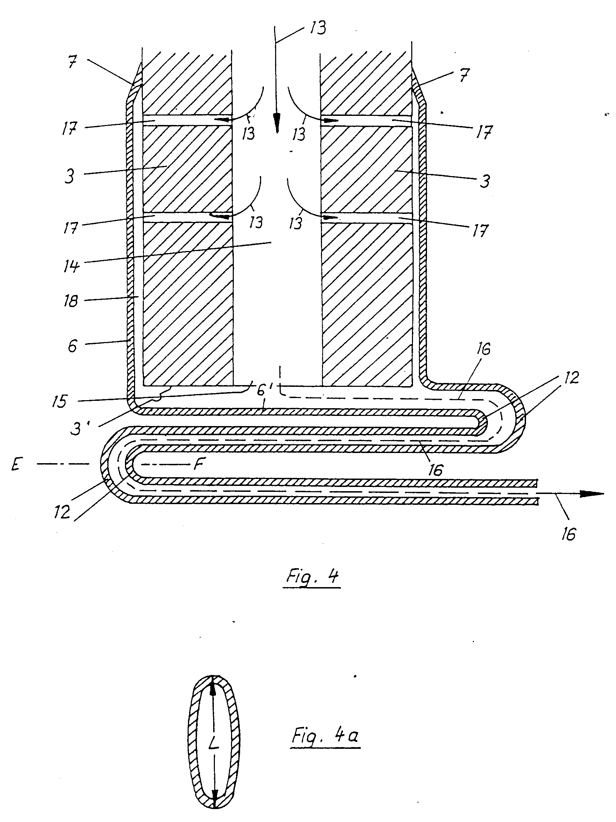

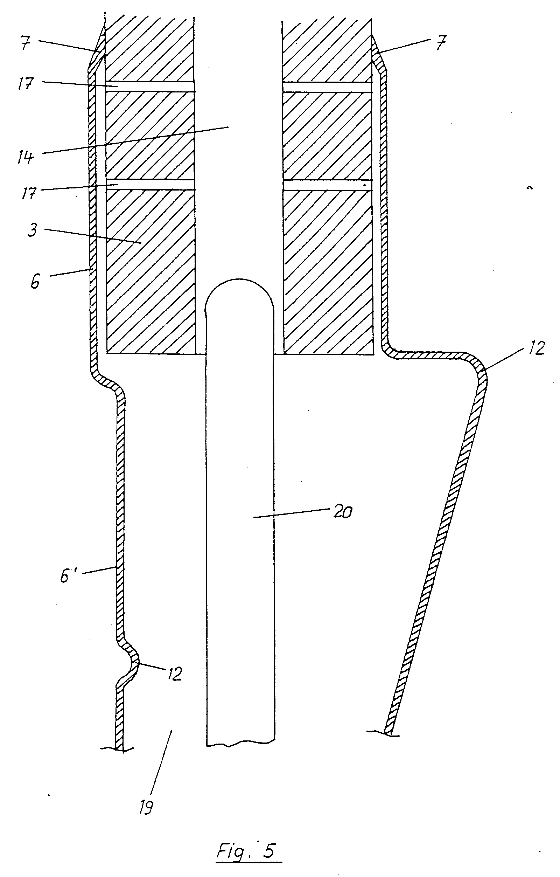

Das Ausführungsbeispiel der Fig. 4 und 5 zeigt einen Teil der Ureterschiene 1 mit dem blasenseitigen Ende 3 und einem Schlauch 6, der ebenfalls gemäß Ziffer 7 dicht an der Außenfläche der Ureterschiene 1 angebracht ist (siehe oben). Das Ziel der Erfindung, auch bei sehr geringen Druckerhöhungen in der Blase für eine Sperre gegen ein Hochsteigen des Urins in der Ureterschiene zu sorgen, ist hier zunächst dadurch gelöst, daß der Schlauch 6 zum einen - ebenso wie im Ausführungsbeispiel der Fig. 1 - über die Stirnfläche 3' des blasenseitigen Endes der Ureterschiene hinausragt und zum andern vorgeprägte Abbiegungen 12 derart aufweist, daß er diese geprägte Zick-Zack-Form beibehält. Hierbei haben die die "Zick-Zack"-Form bildenden, in Fig. 4 waagerecht verlaufenden Bereiche (siehe z.B. Ziffer 6') des Schlauches voneinander und auch von der Stirnfläche 3' der Ureterschiene einen gewissen Abstand. Der Urin kann gemäß Pfeil 13 vom Nierenbecken durch das Lumen 14 der Ureterschiene nach unten durch die Lumenöffnung 15 und von dort, wie mit Pfeil 16 eingezeichnet, in die Blase abfließen. Es können ferner in der Wandung der Ureterschiene, und zwar im Bereich zwischen der Anbringung 7 des Schlauches 6 und der Stirnfläche 3', Drainagekanäle 17 vorgesehen sein, welche das Lumen 14 mit einem schmalen, hohlzylindrischen Raum 18 zwischen Innenfläche des Schlauches 6 und Außenfläche der Ureterschiene 1 verbinden. Somit kann der gemäß Ziffer 13 von der Niere her fließende Urin auch durch diese Drainagekanäle und den hohlzylindrischen Raum 18 abfließen. Ergibt sich aber die zuvor erläuterte Erhöhung des Innendruckes und damit ein den Patienten gefährdendes Hochsteigen des Urins entgegen Pfeilrichtung 13, so bewirkt das entstehende Druckgefälle zum einen, daß sich die in Fig. 4 waagerechten Schlauchabschnitte (z.B. Ziffer 6') gegeneinander und insbesondere gegen die Stirnfläche 3' des blasenseitigen Ureterschienenendes 3 legen. Außerdem bewirkt dieses Druckgefälle, daß sich der Schlauch 6 gegen die nach außen gerichteten Öffnungen der Drainagekanäle 17 legt und diese ebenfalls verschließt. Damit ist das befürchtete Hochsteigen des Urins verhindert.

Die vorstehend erläuterten Drainagekanäle 17 und die Schaffung des hohlzylindrischen Raumes 18 ist bei dieser Ausführungsform der Erfindung nicht unbedingt notwendig, hat aber die erläuterten Vorteile. Die Drainagekanäle 17 und der hohlzylindrische Raum 18 können auch bei der Ausführung nach Fig. 1 vorgesehen sein. Im Falle eines unrunden Querschnittes des Schlauches empfiehlt es sich, daß seine lange Seite L (siehe weiter unten erläuterte Fig. 4a) senkrecht zu den Drainagekanälen 17 verläuft. Hiermit liegen den außenseitigen Öffnungen der Drainagekanäle 17 die langen Seiten des Schlauchquerschnittes gegenüber, wodurch das Anlegen dieser Schlauchseiten an die Außenfläche der Ureterschiene entsprechend erleichtert wird. Ferner sei an dieser Stelle vermerkt, daß der Querschnitt der Ureterschiene kreisringförmig ist, daß aber der in seinem Querschnitt unrunde, z.B. langgestreckte Schlauch für das Überziehen über das Ureterschienenende 3 und Befestigen an der Stelle 7 problemlos in einen entsprechenden, etwa kreisrunden Querschnitt zu bringen ist. Die Ausführungsform nach Fig. 4 ermöglicht ferner durch Entfalten bzw. Auseinanderziehen der in Fig. 4 im Zick-Zack laufenden Schlauchabschnitte, und zwar mit Überwindung der Kraft der eingeprägten "Memory", daß dieser Schlauch 6, 6' gemäß Fig. 5 einen Hohlraum 19 bildet, durch den ein Mandrin 20 in das Lumen 14 der Ureterschiene 1 eingebracht werden kann. Ein solcher Mandrin ist notwendig, um für das Einführen der Ureterschiene in den Ureter und weiter in die in Fig. 1 dargestellte Lage an ihren Enden 2 und 3 unter Überwindung der vorgeprägten Krümmungen zu strecken. Sobald die Ureterschiene die in Fig. 1 dargestellte Endposition erreicht hat, wird der Mandrin 20 herausgezogen und die Ureterschienenenden 2, 3 nehmen ihre vorgeprägte Krümmung, d.h. die in Fig. 1 dargestellte Lage ein.

Vorteilhafterweise kann die Lehre der Ausführung nach Fig. 4 und 5 mit der Lehre, dem Schlauch 6 einen unrunden Querschnitt zu geben, kombiniert werden. Dies zeigt Fig. 4a, die einen Schnitt nach der Linie E-F der Fig. 4 darstellt. Dabei ist die Lage der langen Achse L des Querschnittes so gewählt, daß sie zur Knick- oder Biegelinie des Schlauches parallel verläuft, bevorzugt damit zusammenfällt. Dies erleichtert bei Entstehen eines Überdruckes das Aneinanderlegen der Schlauchwände und das Anliegen des in Fig. 4 obersten Schlauchabschnittes 6' an die Stirnfläche 3'.

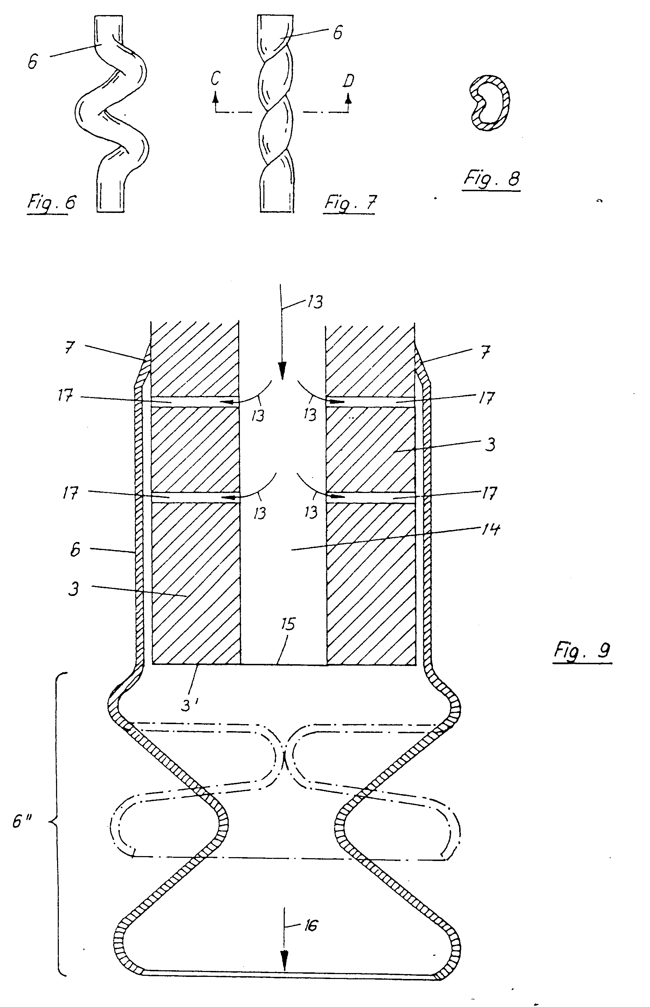

Dem Schlauch 6 kann auch eine korkenzieherförmige (Fig. 6) oder spiralförmige Konfiguration (Fig. 7) eingeprägt werden. Bei Entstehen eines Überdruckes und des o.g. Druckgefälles legen sich die Windungen des "Korkenziehers" oder die Wände der Spirale (siehe hierzu den Querschnitt C-D in Fig. 8) ebenfalls entsprechend zusammen und dichten ab. Auch in diesen Ausführungsbeispielen kann die Schlauchbefestigung und Anordnung an der Ureterschiene so sein, wie in den Fig. 4 und 5 dargestellt. In Fig. 6 und 7 ist der Schlauch im Querschnitt bevorzugt oval oder langgestreckt, wie es auch Fig. 8 zeigt.

Die Lehre der Erfindung gemäß den Fig. 4 bis 8, dem Schlauch eine vor- bzw. eingeprägte Form oder Krümmung im Sinne einer "Memory" zu geben, ließe sich im übrigen bei einem aus zwei Folien zusammengeschweißten Rückschlagventil gemäß der eingangs erörterten DE-OS 35 25 165 nicht oder nur mit Schwierigkeiten verwirklichen, da dies die seitlich verschweißten Folienränder verhindern oder zumindest in der Praxis beeinträchtigen.

In der Ausführungsform gemäß Fig. 9 ist der Schlauch 6 in dem über die Stirnfläche 3' hinausragenden Bereich 6'' mit unterschiedlichen Durchmessern versehen, die sich bei einem entsprechenden hohen Innendruck der Blase zusammenlegen und in etwa die gestrichelte Position einnehmen, in der sie die Öffnung 15 des Lumen 14 abdichten. Die hiermit erzielten Ausbuchtungen können auch die Form einer sogenannten Eieruhr haben. Zugleich wird auch in dieser Ausführung der Schlauch 6 im Bereich der Ureterschiene gegen die Außenöffnungen der Drainagekanäle 17 dichtend angedrückt. Die vorgenannte Form des Schlauches im Bereich 6'' ist ebenfalls in der Fertigung vorgeprägt, d.h. hat eine sogenannte "Memory". Im Normalfall, d.h. bei genügendem Abfluß des Urins aus der Blase, bewirkt die Memory eine Formgebung des Schlauches, welche das Fließen des Urins aus der Niere in Pfeilrichtung 13, 16 in die Blase gestattet. Analoges gilt (nicht im einzelnen dargestellt) für die Varianten gemäß den Fig. 6 bis 8.

## Patentansprüche

1. Rohrförmige Ureterschiene aus einem elastischen Material, insbesondere Kunststoff, die in ihrer Gebrauchslege mit einem ihrer beiden Enden (2, 3) im Nierenbecken und mit ihrem anderen Ende (3) in der Blase gelegen ist, wobei an das zum Einbringen in die Blase vorgesehene Ende (3) außenseitig ein dünnwandiger, weicher und zumindest in seinem Querschnitt einstückiger Schlauch (6) dicht angebracht ist, der über die Stirnfläche dieses Ureterschienenendes hinausragt, dadurch gekennzeichnet, daß der Querschnitt des Schlauches (6) unrund ist.

2. Ureterschiene nach Anspruch 1, dadurch gekennzeichnet, daß der Schlauch (6) einen ovalen oder elliptischen Querschnitt aufweist.

3. Ureterschiene nach Anspruch 1, dadurch gekennzeichnet, daß der Querschnitt des Schlauches (6) etwa die Form eines langgestreckten Rechteckes aufweist, wobei die Übergänge (8) von den langen zu den kurzen Seiten (9, 10) des Querschnittes zumindest etwas abgerundet sind.

4. Rohrförmige Ureterschiene aus einem elastischen Material, insbesondere Kunststoff, die in ihrer Gebrauchslage mit einem ihrer beiden Enden (2, 3) im Nierenbecken und mit ihrem anderen Ende (3) in der Blase gelegen ist, wobei an das zum Einbringen in die Blase vorgesehenen Ende (3) außenseitig ein dünnwandiger, weicher und zumindest in seinem Querschnitt einstückiger Schlauch (6) dicht angebracht ist, der über die Stirnfläche (3') dieses Ureterschienenendes hinausragt, dadurch gekennzeichnet, daß der die Stirnfläche (3') des blasenseitigen Ureterschienenendes (3) überragende Teil des Schlauches (6) eine eingeprägte Form aufweist derart, daß der Schlauch eine Zick-Zack-Form, oder eine Spiralform, oder eine Korkenzieherform aufweist, oder daß sich entlang des überragenden Schlauchabschnittes dessen Durchmesser vergrößert und wieder verkleinert.

5. Rohrförmige Ureterschiene nach Anspruch 1 und 4, gekennzeichnet durch die Kombination der Lehre des Anspruches 1 mit einer der Ausführungen des Anspruches 4.

6. Ureterschiene nach einem der Ansprüche 1 bis 3 und 5, dadurch gekennzeichnet, daß die Lage der langen Achse (L) des unrunden Querschnittes des Schlauches (6) so ist, daß dies das Aufeinanderlegen und Abdichten der zick-zack-förmigen, spiralförmigen, korkenzieherförmigen oder sich im Querschnitt ändernden Wandteile des Schlauches (6) im Fall einer Drucksteigerung im Blaseninnern erleichtert.

7. Ureterschiene nach einem der Ansprüche 1 bis 3, 5 und 6, dadurch gekennzeichnet, daß die Lage der langen Achse (L) des Querschnittes des Schlauches (6) so gewählt ist, daß sie zu Knick- oder Biegelinien des Schlauches parallel verläuft, bevorzugt damit zusammen fällt.

8. Ureterschiene nach einem der Ansprüche 1 bis 7, dadurch gekennzeichnet, daß im blasenseitigen Ende (3) der Ureterschiene (1) von deren Lumen (14) nach außen führende Drainagekanäle (17) vorgesehen sind, die sich im Bereich zwischen der dichten Anbringung (7) des Schlauches (6) an der Ureterschiene und der Stirnfläche (3') des blasenseitigen Ureterschienenendes (3) befinden, wobei zwischen Ureterschienenaußenfläche und Schlauchinnenfläche sich ein schmaler Zwischenraum (18) befindet.

9. Ureterschiene nach Anspruch 8, dadurch gekennzeichnet, daß im Fall eines unrunden Querschnittes des Schlauches (6), dessen lange Seite (L) senkrecht zu den Drainagekanälen (17) verläuft.

10. Ureterschiene nach einem der Ansprüche 4 bis 9, dadurch gekennzeichnet, daß der über seine Längsrichtung mit einer eingeprägten Form versehene Schlauchabschnitt in seiner Längsrichtung so weit auseinanderziehbar ist, daß durch ihn hindurch ein Mandrin (20) in das Lumen (14) der Ureterschiene einbringbar ist.

## Claims

1. Tubular ureter splint composed of an elastic material, in particular plastic, which, in its working position is situated with one of its two ends (2, 3) in the renal pelvis and with its other end (3) in the bladder, wherein there is tightly fitted to the outside of the end (3) provided for introduction into the bladder a thin-walled, soft flexible hose (6) which is integral at least in its cross section and which projects beyond the end face of this ureter splint end, characterized in that the cross section of the flexible hose (6) is noncircular.

2. Ureter splint according to Claim 1, characterized in that the flexible hose (6) has an oval or elliptical cross section.

3. Ureter splint according to Claim 1, characterized in that the cross section of the flexible hose (6) has roughly the shape of an elongated rectangle, wherein the transitions (8) from the long to the short sides (9, 10) of the cross section are at least somewhat rounded.

4. Tubular ureter splint composed of an elastic material, in particular plastic, which, in its working position is situated with one of its two ends (2, 3) in the renal pelvis and with its other end (3) in the bladder, wherein there is tightly fitted to the outside of the end (3) provided for introduction into the bladder a thin-walled, soft flexible hose (6) which is integral at least in its cross section and which projects beyond the end face (3') of this ureter splint end, characterized in that that part of the flexible hose (6) which projects beyond the end face (3') of the bladder-side ureter splint end (3) has an impressed shape which is such that the flexible hose has a zigzag shape or a spiral shape or a corkscrew shape or that, along the projecting section of the flexible hose, its diameter increases and decreases again.

5. Tubular ureter splint according to Claims 1 and 4, characterized by the combination of the teaching of Claim 1 with one of the embodiments of Claim 4.

6. Ureter splint according to one of Claims 1 to 3 and 5, characterized in that the position of the long axis (L) of the noncircular cross section of the flexible hose (6) is such that it facilitates the superposition and sealing of the zigzag-shaped, spiral-shaped, corkscrew-shaped wall parts of the flexible hose (6) or wall parts of the flexible hose (6) which change in cross section in the event of an increase in pressure in the interior of the bladder.

7. Ureter splint according to one of Claims 1 to 3, 5 and 6, characterized in that the position of the long axis (L) of the cross section of the flexible hose (6) is chosen in such a way that it extends parallel to the inflection line or bending line of the flexible hose, and preferably coincides therewith.

8. Ureter splint according to one of Claims 1 to 7, characterized in that there are provided in the bladder-side end (3) of the ureter splint (1) drainage ducts (17) which lead outwards from its lumen (14) and which are situated in the region between the tight fitting (7) of the flexible hose (6) to the ureter splint and the end face (3') of the bladder-side ureter splint end (3), a narrow gap (18) being situated between the outside surface of the ureter splint and the inside surface of the flexible hose.

9. Ureter splint according to Claim 8, characterized in that, if the cross section of the flexible hose (6) is noncircular, its long side (L) extends perpendicularly to the drainage ducts (17).

10. Ureter splint according to one of Claims 4 to 9, characterized in that the flexible hose section provided over its longitudinal direction with an impressed shape can be pulled apart in its longitudinal direction to such an extent that a mandrin (20) can be brought through it into the lumen (14) of the ureter splint.

## Revendications

1. Conduit urétéral tubulaire, fait d'un matériau élastique, en particulier de matière plastique, qui, en sa position d'utilisation, est situé par l'une de ses deux extrémités (2, 3) dans le bassinet du rein et par son autre extrémité (3) dans la vessie, avec disposition étanche, sur le côté extérieur de l'extrémité (3) prévue pour être introduite dans la vessie, d'un tuyau (6) à paroi mince, souple et au moins d'un seul tenant en sa section droite, qui dépasse de la face frontale de cette extrémité du conduit urétéral caractérisé en ce que la section droite du tuyau (6) n'est pas circulaire.

2. Conduit urétéral selon la revendication 1, caractérisé en ce que le tuyau (6) possède une section droite ovale ou elliptique.

3. Conduit urétéral selon la revendication 1, caractérisé en ce que la section droite du tuyau (6) possède à peu près la forme d'un rectangle oblong dont les transitions (8) entre les grands côtés (9) et les petits côtés (10) de la section droite sont au moins un peu arrondis.

4. Conduit urétéral tubulaire, fait d'un matériau élastique, en particulier de matière plastique, qui, en sa position d'utilisation, est situé par l'une de ses deux extrémités (2, 3) dans le bassinet du rein et par son autre extrémité (3) dans la vessie, avec disposition étanche, sur le côté extérieur de l'extrémité (3) prévue pour être introduite dans la vessie, d'un tuyau (6) à paroi mince, souple et au moins d'un seul tenant en sa section droite, qui dépasse de la face frontale (3') de cette extrémité du conduit urétéral , caractérisé en ce que la partie du tuyau (6) dépassant de la face frontale (3') de l'extrémité vésicale (3) du conduit urétéral possède une forme qui lui a été imposée, de telle sorte que le tuyau présente une forme en zigzag, hélicoïdale ou en tire-bouchon, ou que le diamètre de la partie de tuyau dépassante augmente et diminue de nouveau le long de cette partie de tuyau.

5. Conduit urétéral tubulaire selon les revendications 1 et 4, caractérisé par la combinaison de l'enseignement apporté par la revendication 1 avec l'une des exécutions de la revendication 4.

6. Conduit urétéral selon une des revendications 1 à 3 et 5, caractérisé en ce que la position du grand axe (L) de la section droite non circulaire du tuyau (6) est telle qu'elle facilite l'application l'une contre l'autre et la fermeture étanche des portions de paroi de forme en zigzag, hélicoïdale, en tire-bouchon ou de section droite changeante du tuyau (6) dans le cas d'une élévation de la pression à l'intérieur de la vessie.

7. Conduit urétéral selon une des revendications 1 à 3, 5 et 6, caractérisé en ce que la position du grand axe (L) de la section droite du tuyau (6) est choisie de manière qu'elle soit parallèle à des lignes de pliage ou de courbure du tuyau et coïncide de préférence avec elles.

8. Conduit urétéral selon une des revendications 1 à 7, caractérisé en ce que l'extrémité vésicale (3) du conduit urétéral (1) présente des canaux de drainage (17) qui mènent de la lumière (14) de cette extrémité vers l'extérieur et se trouvent entre la disposition étanche (7) du tuyau (6) sur le conduit et la face frontale (3') de l'extrémité vésicale (3) du conduit, avec prévision d'un étroit espace intermédaire (18) entre la surface externe du conduit urétéral et la surface interne du tuyau.

9. Conduit urétéral selon la revendication 8, caractérisé en ce que, au cas où le tuyau (6) possède une section droite non circulaire, le grand axe (L) de cette section est orienté transversalement aux canaux de drainage (17).

10. Conduit urétéral selon une des revendications 4 à 9, caractérisé en ce que le tronçon de tuyau longitudinal pourvu d'une forme imposée, est extensible dans le sens de sa longueur au point qu'un mandrin (20) peut être introduit à travers lui dans la lumière (14) du conduit.
